# FASCICULE DE BREVET EUROPEEN

(11) **EP 1 982 746 B1**
(45) Date de publication et mention de la délivrance du brevet: **07.04.2010**
(21) Numéro de dépôt: 08290339.4
(22) Date de dépôt: 08.04.2008
(51) Int. Cl.: A61N 1/372, G06F 19/00, G06Q 50/00, G06F 9/445

(54) **Programmateur pour implant cardiaque, comportant un mode de test accéléré des paramètres**
Programmierer für Herzimplantat mit beschleunigtem Testmodus zum Testen der Parameter
Programmer for a cardiac implant, comprising an accelerated parameter test mode

(30) Priorité: 19.04.2007 FR 0702826
(43) Date de publication de la demande: 22.10.2008
(73) Titulaire: ELA MEDICAL, 92541 Montrouge (FR)
(72) Inventeur: Catto, Giovanni, 94250 Gentilly (FR)
(74) Mandataire: Dupuis-Latour, Dominique

(56) Documents cités:
- US-A- 5 833 623
- US-A1- 2004 143 304
- US-B1- 6 618 622
- US-B1- 7 003 349

## Description

L'invention concerne les programmateurs externes destinés à être couplés à des "dispositifs médicaux implantables actifs" tels que définis par la directive 90/385/CEE du 20 juin 1990 du Conseil des communautés européennes, plus précisément des implants cardiaques de stimulation, de resynchronisation, de cardioversion et/ou de défibrillation.

Le programmateur permet d'interroger l'implant pour lire le contenu des mémoires de celui-ci, le programmer pour en modifier les réglages, ou de piloter l'exécution de divers algorithmes de mise à jour du logiciel, de test de fonctionnement, etc.

Une fois l'implant mis en place et ses sondes raccordées, il est nécessaire de vérifier un certain nombre de paramètres au moment de l'implantation, puis régulièrement au cours de visites de contrôle.

Les principaux paramètres à tester sont : la sensibilité de détection, l'impédance de sonde et le seuil de capture. Ces tests doivent être effectués à la fois pour le ventricule et l'oreillette. Dans le cas d'un défibrillateur, le test porte également sur la continuité du bobinage ou de l'électrode de choc, et sur le temps de charge du condensateur de choc jusqu'à sa valeur nominale d'énergie. Enfin, pour les dispositifs multisite, notamment les implants de resynchronisation, il est nécessaire de tester en outre les paramètres relatifs au ventricule gauche (impédance de la sonde coronaire de stimulation gauche, et seuil de capture de l'électrode ventriculaire gauche).

L'exécution de ces tests est commandée par le praticien, à partir du programmateur.

Les US 7 003 349 B1, US 6 618 622 B1 et US 5 833 623 décrivent des dispositifs adaptés à l'exécution de tels tests. Le praticien peut en particulier disposer de "scripts" définissant une séquence d'opérations où s'enchaînent automatiquement, l'un après l'autre, les différents tests demandés.

Lorsque le praticien demande l'exécution d'un test, ceci a pour effet de placer l'implant dans un réglage particulier de son mode de fonctionnement (DDI, DOO, ... ), de la fréquence de stimulation (valeur choisie par le praticien ou fixée par défaut par le programmateur) et du délai atrioventriculaire. Le programmateur recueille ensuite les données de fonctionnement de l'implant avec ces réglages prédéterminés, et affiche sur un écran le résultat du test ainsi effectué.

Ces tests peuvent être commodément lancés par appui sur un bouton à disposition du praticien (bouton physique, ou zone à cliquer sur un écran), chaque appui du bouton déclenchant le test correspondant.

Le temps nécessaire à l'exécution des différents tests est une donnée importante.

En premier lieu, il est souhaitable d'abréger la phase des tests, car il s'agit là d'un préalable à toute interprétation ou diagnostic, et donc d'un temps mort sur la durée de la consultation par le praticien.

Surtout, du point de vue du patient, pendant les tests l'implant est ajusté avec des réglages prédéterminés spécifiques aux tests, et son fonctionnement n'est pas celui pour lequel il est normalement paramétré. L'implant n'est donc pas adapté à la physiologie du patient pendant la durée de tous ces tests. Il est donc extrêmement souhaitable, par sécurité, d'abréger autant que possible cette phase de tests.

L'un des buts de l'invention est de permettre une réduction notable de la durée nécessaire à l'exécution des différents tests de fonctionnement de l'implant.

On verra ainsi que, grâce à l'invention, la durée totale des tests d'un stimulateur peut être réduite à moins d'une minute, alors qu'en pratique cette durée n'est aujourd'hui jamais inférieure à 2 min 15 ou 2 min 30.

Un autre but de l'invention est de proposer au praticien l'exécution d'actions spécifiques enchaînées automatiquement après exécution des étapes de tests, sans que l'opérateur n'ait à intervenir : mémorisation des résultats, impression d'un compte rendu, activation d'un module d'aide au diagnostic, ...

L'idée de base de l'invention consiste à opérer une compression temporelle de la séquence de tests par enchaînements automatiques des différents tests avec chevauchements (exécution simultanée) de certains d'entre eux.

Le programmateur de l'invention comprend, de manière en elle-même connue (par exemple d'après le US 7 003 349 B1 précité) des moyens de télémétrie, pour un couplage bidirectionnel avec l'implant, et une interface utilisateur, pour définir des tests à effectuer sur l'implant et pour afficher les résultats de ces tests. Ces tests incluant au moins certains des tests du groupe : sensibilité de détection ventriculaire, sensibilité de détection auriculaire, impédance de sonde ventriculaire, impédance de sonde auriculaire, seuil de capture ventriculaire, seuil de capture auriculaire. le programmateur comprend également des moyens de commande pour exécuter une pluralité d'étapes de test, chaque étape comprenant (i) un réglage prédéterminé du mode, de la fréquence de stimulation et du délai atrioventriculaire de l'implant, (ii) le recueil des données de fonctionnement de l'implant selon ces réglages prédéterminés, et (iii) le traitement et l'affichage des données ainsi recueillies.

De façon caractéristique de l'invention, la pluralité d'étapes de test comprend au moins une étape à compression temporelle au cours de laquelle au moins certains des tests ventriculaire et auriculaire d'un même paramètre sont exécutés en parallèle au cours d'une étape commune, de façon non séquentielle, ni alternative.

Les tests en question sont concomitants, c'est-à-dire exécutés en parallèle au cours d'un même laps de temps, par opposition à une exécution qui serait opérée "séquentiellement" ou "alternativement".

Diverses caractéristiques subsidiaires sont indiquées dans les sous-revendications.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 illustre un exemple d'écran constituant l'interface utilisateur du programmateur selon l'invention, pour l'exécution des tests de fonctionnement d'un stimulateur cardiaque.

La figure 2 est un organigramme montrant l'enchaînement des différentes étapes de tests susceptibles d'être commandés par le praticien au moyen de l'interface de la figure 1.

La figure 3 illustre un exemple d'écran constituant l'interface utilisateur du programmateur selon l'invention, pour l'exécution des tests de fonctionnement d'un dispositif multisite incluant des fonctions de resynchronisation et/ou de défibrillation.

La figure 4 est un organigramme montrant l'enchaînement des différentes étapes de tests susceptibles d'être commandés par le praticien au moyen de l'interface de la figure 3.

On va maintenant décrire un exemple de réalisation du dispositif de l'invention.

En ce qui concerne ses aspects logiciels, l'invention peut être mise en oeuvre par une programmation appropriée du logiciel de commande d'un programmateur connu.

L'invention peut notamment être appliquée aux programmateurs *REPLY* et *PARADYM* commercialisés par ELA Médical, Montrouge, France, destinés à la programmation de dispositifs tels que les appareils *SYNERGY* et

*SYNDELI* de cette même société. Ces programmateurs sont des microordinateurs configurés spécifiquement pour mettre en oeuvre les fonctions de programmation, en liaison avec une tête de télémétrie connectée à l'ordinateur et couplée à l'implant par voie inductive ou radiofréquence. L'adaptation de ces appareils à la mise en oeuvre des fonctions de l'invention est à la portée de l'homme du métier, et elle ne sera pas décrite en détail.

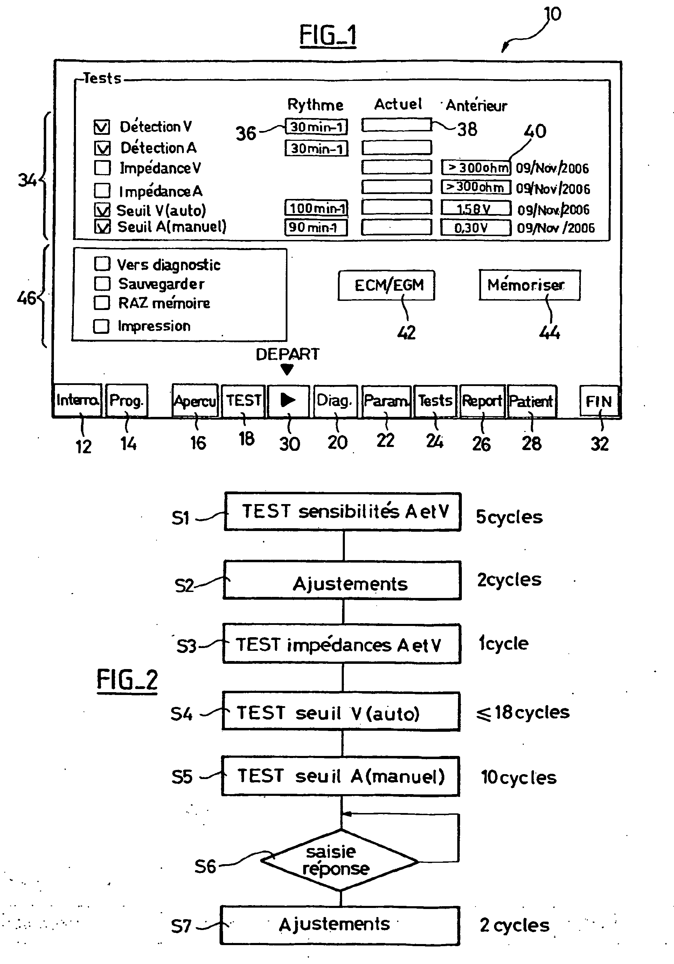

Sur la figure 1, on a illustré en 10 l'interface présentée au praticien sur l'écran du programmateur.

De manière en elle-même connue, cette interface comporte un bouton 12 de lecture pour commander l'interrogation des mémoires et des registres de l'implant, et un bouton d'écriture 14 pour la programmation des paramètres de fonctionnement de celui-ci. L'écran 10 comporte en outre un certain nombre de boutons 16, 18, 20, 22, 24, 26, 28 permettant de sélectionner des fonctions telles que : aperçu de l'état de l'implant, déclenchement des tests, activation d'un module d'aide au diagnostic, paramétrage, vérification, compte rendu, identification du patient, ...

Au bouton 18 de déclenchement des tests est associé un bouton "départ" 30 destiné à exécuter, de la manière spécifique de l'invention, les différents tests de fonctionnement de l'implant. Une touche "fin" 32 permet de terminer la session de suivi.

L'écran comporte un certain nombre de cases à cocher 34, permettant au praticien de sélectionner les tests qu'il souhaite, ou non, exécuter : sensibilité de détection ventriculaire, sensibilité de détection auriculaire, impédance de sonde ventriculaire, impédance de sonde auriculaire, seuil de capture ventriculaire (ce seuil peut être déterminé automatiquement par l'implant) seuil de capture auriculaire (ce seuil est généralement déterminé manuellement, car il nécessite une interprétation des données par le praticien).

Pour les tests de sensibilité de détection et de seuil de capture, un champ 36 indique à quelle fréquence sera réglé le stimulateur ; la valeur par défaut peut être éventuellement modifiable par le praticien.

Des champs 38 permettent l'affichage des résultats des tests respectifs après exécution de ceux-ci, et des champs 40 indiquent le résultat du test antérieurement effectué par l'implant pour ce même paramètre, avec la date correspondante. En effet, les valeurs de certains paramètres, notamment l'impédance et le seuil de capture, sont mesurées automatiquement par l'implant de façon autonome et à intervalles réguliers (par exemple toutes les cinq heures). Ce sont ces valeurs qui sont affichées dans les champs 40.

Un bouton 42 permet de revoir a posteriori l'électrocardiogramme (ECG) et l'électrogramme (EGM) associés à l'exécution des tests, et un bouton 44 permet de conserver dans une mémoire du programmateur les résultats des tests (la mémorisation dans l'implant faisant l'objet d'une autre commande).

L'interface 10 comporte également un certain nombre de cases à cocher 46 permettant au praticien de demander l'exécution d'actions spécifiques par le programmateur immédiatement après achèvement de la séquence de tests : activation d'un module d'aide au diagnostic, sauvegarde dans une mémoire de l'implant des résultats des tests, remise à zéro de cette mémoire, impression d'un compte rendu.

La séquence des différents tests est schématisée sur l'organigramme de la figure 2. La séquence illustrée correspond à l'exécution de la totalité des tests qu'il est possible de sélectionner par les cases à cocher 34, mais si certaines de ces cases n'étaient cochées, bien entendu les étapes correspondantes seraient omises lors du déroulement de la séquence.

La première étape S1 consiste à évaluer la sensibilité de détection auriculaire et ventriculaire. Ces deux mesures sont effectués simultanément au cours de cette même étape S1. Typiquement, les réglages de l'implant sont : mode DDI, délai atrioventriculaire (DAV) 250 ms, fréquence 30 bpm par défaut (ou autre valeur programmée par le praticien).

Cette étape peut être exécutée en cinq cycles seulement, soit une durée de 10 secondes à une fréquence de 30 bpm.

L'étape suivante S2 est une étape d'ajustement permettant de compenser les modifications du mode de fonctionnement, du DAV et de la fréquence entre l'étape S1 et l'étape suivante S3. Dans cette étape d'ajustement S2, les réglages de l'implant deviennent : mode DOO, DAV programmé, fréquence 100 bpm. La durée typique est de deux cycles.

L'étape suivante S3 est une étape de mesure des impédances ventriculaire et auriculaire. Avec les réglages opérés à l'étape précédente (mode DOO, DAV programmé, fréquence 100 bpm), ce test peut être effectué en un cycle seulement, simultanément pour les électrodes ventriculaire et auriculaire. Il est notamment possible de mettre en oeuvre une technique que celle décrite dans le EP-A-1 216 723 (Ela Medical).

L'étape suivante S4 est une étape de détermination du seuil de capture ventriculaire. Les réglages de l'implant sont : mode DOO, DAV 94 ms, fréquence 100 bpm par défaut (ou autre valeur programmée par le praticien). Cette étape S4 se décompose en une première phase de calibration, typiquement sur sept cycles, suivie d'une phase de test de capture avec des impulsions d'amplitude décroissante. Très avantageusement, le test est considéré comme terminé dès qu'une perte de capture est détectée, ce qui permet de raccourcir d'autant la durée de cette étape. La durée maximale de cette étape S4 est de 18 cycles (dans l'hypothèse la moins favorable, où la perte de capture n'est pas détectée, ou n'est détectée qu'au dernier cycle). Il est notamment possible de mettre en oeuvre une technique que celle décrite dans le EP-A-1 080 744 (Ela Medical), avec une phase de calibration suivie d'une phase de recherche de la perte de capture.

L'étape suivante S5 est une étape de mesure du seuil de capture auriculaire. Bien qu'il existe des techniques de mesure automatique de ce seuil, telle que celle décrite par exemple dans le EP-A-1 743 675 (Ela Medical), il peut être préférable, par sécurité, de recourir à une interprétation du praticien.

Pour ce faire, le stimulateur est piloté sur dix cycles avec les paramètres suivants : mode DAO, DAV 250 ms, fréquence 100 bpm par défaut (ou autre valeur programmée par le praticien). À l'issue de ce test, le praticien indique (étape S6) la valeur de seuil de capture auriculaire suite à son interprétation des signaux cardiaques qui lui ont été affichés sur un écran intermédiaire.

Les différents tests ainsi achevés, la séquence s'achève par une étape S7 de deux cycles d'ajustement, pour replacer le stimulateur dans sa configuration d'origine, telle qu'elle était avant le test.

On notera que les étapes d'ajustement S2 et S7 sont nécessaires pour permettre un enchaînement automatique de toutes les étapes successives, à la différence des configurations connues où les tests étaient commandés les uns après les autres par le praticien, par exemple par des appuis successifs sur un bouton : dans ce cas, les tests étaient exécutés isolément, interprétés visuellement, et les réglages déterminés pour chacun des tests, à chaque fois, sans considération des réglages du test antérieur. Au contraire, dans l'invention, les réglages d'un test donné dépendent des réglages du test précédent, et c'est pour cette raison que, par exemple pour enchaîner les tests S1 de sensibilité de détection et S3 d'impédance, il est nécessaire de prévoir une étape intermédiaire de compensation S2 pour réaliser le changement de mode (de DDI en DOO) et de fréquence de stimulation (de 30 bpm à 100 bpm) de façon entièrement automatique).

Avec les nombres de cycles et fréquences de stimulation indiqués plus haut, on aboutit à un bilan global de 45 secondes au plus pour les étapes S1-S5 et S7 (la durée du test de seuil de capture ventriculaire pouvant être souvent abrégée, comme expliqué plus haut). Si l'on prévoit une durée moyenne de 10 secondes pour l'étape S6 d'interprétation du résultat du test de capture auriculaire et du choix de la valeur de seuil par le praticien, on aboutit à une durée totale, pour l'ensemble des étapes S1-S7, de 55 secondes au maximum - à comparer aux durées habituelles de 2 min 15 à 2 min 30 avec une séquence test manuelle.

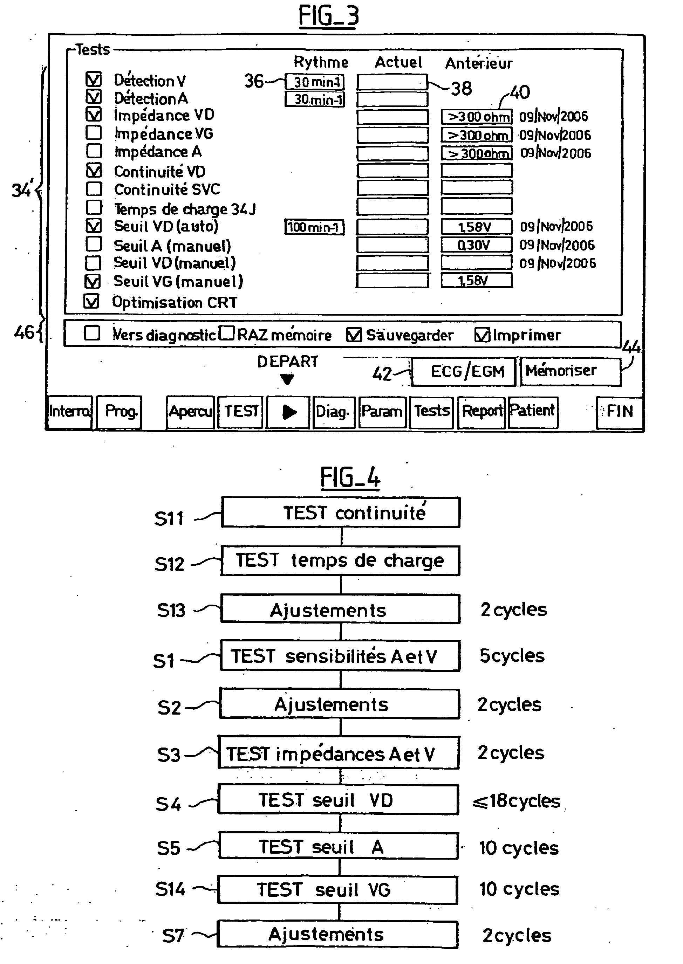

La figure 3 illustre l'interface présentée au praticien pour le test d'un dispositif multisite incluant des fonctions de resynchronisation et/ou de défibrillation/cardioversion.

On y retrouve en 34' les cases à cocher que l'on avait avec l'interface de la figure 1, plus un certain nombre d'autres paramètres à tester, notamment (en fonction du type d'implant) : impédance de sonde ventriculaire gauche, continuité du bobinage de choc (électrode dite "supraventriculaire" et/ou électrode ventriculaire distale), temps de charge du condensateur de choc à son énergie nominale, seuil de capture ventriculaire gauche, éventuellement seuil de capture ventriculaire droit en mode manuel (pour tester manuellement, en les comparant, les seuils de capture pour les deux cavités ventriculaires), et test d'optimisation d'une thérapie de resynchronisation (CRT).

Pour le reste, les informations présentées sur l'écran sont les mêmes que dans le cas de la figure 1.

La figure 4 illustre l'enchaînement des différentes étapes de test.

Outre les étapes S1 à S7 décrites à propos de la figure 2, la séquence comprend les étapes supplémentaires suivantes :
- étape S11 de test de continuité du ou des bobinages de défibrillation,
- étape S12 de test du temps de charge,
- étape S13 d'ajustement (deux cycles) pour replacer le stimulateur avec ses valeurs programmées de mode, de DAV, de fréquence et d'amplitude,
- étape S14 de test de seuil ventriculaire gauche, avec les paramètres : mode DAO, DAV 94 ms, fréquence 100 bpm par défaut (ou autre valeur programmée par le praticien). La durée de cette étape S14 est de 10 cycles.

Ici encore, l'enchaînement automatique des différentes étapes, et le chevauchement des tests auriculaire et ventriculaire, permet un gain de temps considérable par rapport à une séquence opérée de façon traditionnelle.

## Revendications

1. Un programmateur pour un implant cardiaque de stimulation, de resynchronisation, de cardioversion et/ou de défibrillation, ce programmateur comprenant :
- des moyens de télémétrie, pour un couplage bidirectionnel avec l'implant,
- une interface utilisateur, pour définir des tests à effectuer sur l'implant et pour afficher les résultats de ces tests,
ces tests incluant au moins certains des tests du groupe : sensibilité de détection ventriculaire, sensibilisé de détection auriculaire, impédance de sonde ventriculaire, impédance de sonde auriculaire, seuil de capture ventriculaire, seuil de capture auriculaire,
- des moyens de commande pour exécuter une pluralité d'étapes de test, chaque étape comprenant (i) un réglage prédéterminé du mode, de la fréquence de stimulation et du délai atrioventriculaire de l'implant, (ii) le recueil des données de fonctionnement de l'implant selon ces réglages prédéterminés, et (iii) le traitement et l'affichage des données ainsi recueillies,
**caractérisé en ce que** ladite pluralité d'étapes de test comprend au moins une étape à compression temporelle au cours de laquelle au moins certains des tests (S1, S3) ventriculaire et auriculaire d'un même paramètre sont exécutés en parallèle au cours d'une étape commune, de façon non séquentielle ni alternative.

2. Le programmateur de la revendication 1, dans lequel l'interface utilisateur comprend des moyens (34 ; 34') de sélection préalable par l'utilisateur des tests à effectuer, et les moyens de commande sont aptes à enchaîner en séquence, sans intervention de l'utilisateur, l'exécution des étapes de test correspondantes (S1-S7).

3. Le programmateur de la revendication 1, dans lequel les moyens de commande sont également aptes à exécuter au moins une étape d'ajustement (S2) intercalée entre deux étapes de test (S1, S3) impliquant un changement du mode et/ou de la fréquence de stimulation de l'implant.

4. Le programmateur de la revendication 1, dans lequel les moyens de commande sont également aptes à exécuter au moins une étape (S7) d'ajustement du mode et/ou de la fréquence de stimulation et/ou du délai atrioventriculaire de l'implant après exécution de la dernière étape de test (S5, S6 ; S14).

5. Le programmateur de la revendication 1, dans lequel les moyens de commande sont aptes à commander l'exécution d'au moins :
- une étape (S1) de test simultané de sensibilité de détection ventriculaire et auriculaire, sur N₁ cycles,
- une étape (S3) de test simultané d'impédance de sonde ventriculaire et auriculaire, sur N₂ cycles,
- une étape (S4) de test de seuil de capture ventriculaire, sur N₃ cycles,
- une étape (S5) de test de seuil de capture auriculaire, sur N₄ cycles.

6. Le programmateur de la revendication 5, dans lequel l'étape (S1) de test simultané de sensibilité de détection ventriculaire et auriculaire est exécutée sur N₁ = 5 cycles.

7. Le programmateur de la revendication 5, dans lequel l'étape (S3) de test simultané d'impédance de sonde ventriculaire et auriculaire est exécutée sur N₂ = 1 cycle.

8. Le programmateur de la revendication 5, dans lequel l'étape (S4) de test de seuil de capture ventriculaire est exécutée sur N₃ = N₃' + N₃" cycles, N₃ étant un nombre fixe de cycles de calibration et N₃" étant un nombre variable de cycles de test de capture avec des impulsions d'amplitudes successives décroissantes, les moyens de commande étant aptes à mettre fin au test de seuil de capture ventriculaire dès détection de la perte de capture.

9. Le programmateur de la revendication 8, dans lequel N₃ ≤ 18 cycles.

10. Le programmateur de la revendication 1, dans lequel les moyens de commande sont en outre aptes à commander l'exécution d'au moins :
- une étape (S11) de test de continuité de bobinage de choc,
- une étape (S12) de test de temps de charge d'un condensateur de choc,
- des étapes distinctes (S4, S14) de test de seuil de capture ventriculaire droite et gauche.

11. Le programmateur de la revendication 1, dans lequel l'interface utilisateur comprend des moyens (46) de sélection préalable par l'utilisateur d'au moins une action spécifique à enchaîner en séquence, sans intervention de l'utilisateur, après exécution des étapes de test.

12. Le programmateur de la revendication 11, dans lequel lesdites actions spécifiques sont des actions du groupe comprenant : mémorisation des résultats des tests dans l'implant, mémorisation des résultats des tests dans le programmateur, impression des résultats des tests, activation d'un module d'aide au diagnostic suite aux tests.

## Claims

1. A programmer for a cardiac stimulation, resynchronization, cardioversion and/or defibrillation implant, said programmer comprising :
- telemetry means for a bidirectional coupling with the implant,
- a user interface for defining tests to be done on the implant and displaying results of these tests, said tests including at least some of those belonging to the group consisting of : ventricular detection sensibility, atrial detection sensibility, ventricular lead impedance, atrial lead impedance, ventricular capture threshold, atrial capture threshold,
- control means for performing a plurality of test steps, wherein each step comprises (i) predeterminedly adjusting the mode, the stimulation frequency and the implant atrioventricular delay ; (ii) collecting implant operation data according to above predetermined adjustments, and (iii) processing and displaying data collected as above,
**characterized in that** said plurality of test steps comprises at least one time compression step during which at least some of the ventricular and atrial tests (S1, S3) of same parameter are performed in parallel during a common step in a non-sequential and non-alternative manner.

2. The programmer according to Claim 1, wherein the user interface comprises means (34, 34') allowing the user to proceed to a preliminary selection of tests to be performed, and control means are adapted to perform successively, sequentially, without an intervention of the user, corresponding test steps (S1-S7).

3. The programmer according to Claim 1, wherein the control means also are adapted to perform at least one adjustment step (S2) inserted between two test steps (S1, S3) involving a change of the stimulation mode and/or frequency of the implant.

4. The programmer according to Claim 1, wherein the control means also are adapted to perform at least one step (S7) for adjusting the stimulation mode and/or frequency and/or the atrioventricular delay of the implant after performing the last test step (S5, S6 ; S14).

5. The programmer according to Claim 1, wherein the control means are adapted to perform at least :
- one step (S1) for simultaneously testing the ventricular and the atrial detection sensibilities on N₁ cycles,
- one step (S3) for simultaneously testing the ventricular and the atrial lead impedances on N₂ cycles,
- one step (S4) for testing the ventricular capture threshold on N₃ cycles,
- one step (S5) for testing the atrial capture threshold on N₄ cycles.

6. The programmer according to Claim 5, wherein the step (S1) for simultaneously testing the ventricular and the atrial detection sensibilities is performed on N₁ = 5 cycles.

7. The programmer according to Claim 5, wherein the step (S3) for simultaneously testing the ventricular and the atrial lead impedances is performed on N₁ = 1 cycle.

8. The programmer according to Claim 5, wherein the step (S4) for testing the ventricular capture threshold is performed on N₃ = N_{3'} + N_{3"} cycles, wherein N_{3'} is a fixed number of calibration steps and N_{3"} is a variable number of capture test cycles with pulses having decreasing successive amplitudes, wherein the control means are adapted to terminate the ventricular capture threshold test as soon the capture loss is detected.

9. The programmer according to Claim 8, wherein N₃ ≤ 18 cycles.

10. The programmer according to Claim 1, wherein the control means also are adapted to control the performance of at least :
- one step (S11) for testing the continuity of a shock coil,
- one step (S12) for testing the load time of a shock capacitor,
- distinct steps (S4, S14) for testing the right and the left ventricular capture thresholds.

11. The programmer according to Claim 1, wherein the user interface comprises means (46) allowing the user to preliminarily select at least one specific action to be sequentially performed without an intervention of the user after performing the test steps.

12. The programmer according to Claim 11, wherein said specific actions are actions belonging to the group comprising : storing the test results in the implant, storing the test results in the programmer, printing the test results, activating a diagnosis aid module as a result of the tests.

## Patentansprüche

1. Programmierer für ein Stimulations-, Resynchronisations-, Kardioversions- und/oder Defibrillations-Herzimplantat aufweisend :
- Telemetriemittel für eine bidirektionale Kopplung mit dem Implantat,
- eine Anwenderschnittstelle, um auf das Implantat durchzuführende Tests zu bestimmen und die Ergebnisse dieser Tests anzuzeigen, wobei obengenannte Tests wenigstens einige der Tests folgender Gruppe einschliessen : Empfindlichkeit der Kammerdetektion, Empfindlichkeit der Vorhofdetektion, Impedanz der Kammersonde, Impedanz der Vorhofsonde, Kammerreizschwelle, Vorhofreizschwelle,
- Steuermittel, um eine Vielzahl von Testschritten durchzuführen, aufweisend : eine vorgegebene Regelung des Modus, der Stimulationsfrequenz und der atrioventrikularen Verzögerung des Implantats, (ii) die Sammlung der Betriebsdaten des Implantats in Abhängigkeit von obengenannten vorgegebenen Regelungen, und (iii) die Verarbeitung und das Anzeigen der so gesammelten Daten,
**dadurch gekennzeichnet, dass** genannte Vielzahl von Testschritten wenigstens einen Zeitkompressionsschritt, aufweist, während dessen wenigstens einige der durchzuführenden ventrikularen und aurikularen Tests (S1, S3) desselben Parameters parallel während eines gemeinsamen Schritts in einer nichtsequentiellen und nichtalternativen Weise durchgeführt werden.

2. Der Programmierer gemäss Anspruch 1, in welchem die Anwenderschnittstelle Mittel (34 ; 34') aufweist, um dem Anwender zu erlauben, vorläufig die durchzuführenden Test auszuwählen, und die Steuermittel geeignet sind, die entsprechenden Testschritten (S1-S7) ohne Einsatz des Anwenders sequentiell nacheinander durchzuführen.

3. Der Programmierer gemäss Anspruch 1, in welchem die Steuermittel auch geeignet sind, wenigstens einen zwischen beiden, eine Veränderung des Modus und/oder der Stimulationsfrequenz des Implantats hineinziehenden Testschritten (S1, S3) zwischengeschalteten Anpassungsschritt (S2) durchzuführen.

4. Der Programmierer gemäss Anspruch 1, in welchem die Steuermittel auch geeignet sind, wenigstens einen Anpassungssschritt (S7) des Modus und/oder der Stimulationsfrequenz und/oder der atrioventrikularen Verzögerung nach der Durchführung des letzten Testschritts (S5, S6 ; S14) durchzuführen.

5. Der Programmierer gemäss Anspruch 1, in welchem die Steuermittel geeignet sind, die Durchführung wenigstens
- eines gleichzeitigen Testschritts der Empfindlichkeit der Kammer- und Vorhofdetektion auf N₁ Zyklen,
- eines gleichzeitigen Testschritts (S3) der Impedanz der Vorhof- und Kammer-Sonde auf N₂ Zyklen,
- eines Testschritts (S4) der Kammerreizschwelle auf N₃ Zyklen,
- eines Testschritts (S5) der Vorhofreizschwelle auf N₄ Zyklen,
zu steuern.

6. Der Programmierer gemäss Anspruch 5, in welchem der gleichzeitige Testschritt (S1) der Empfindlichkeit der Kammer- und Vorhofdetektion auf N₁ = 5 Zyklen durchgeführt wird.

7. Der Programmierer gemäss Anspruch 5, in welchem der gleichzeitige Testschritt (S3) der Impedanz der Vorhof- und Kammer-Sonde auf N₂ = 1 Zyklus durchgeführt wird.

8. Der Programmierer gemäss Anspruch 5, in welchem der Testschritt (S4) der Kammerreizschwelle auf N₃ = N_{3'} + N_{3"} Zyklen durchgeführt wird, wobei N_{3'} eine feste Zahl der Kalibrierzyklen und N_{3"} eine veränderliche Zahl der Reiztests mit abnehmenden aufeinanderfolgenden Amplitudenpulsen darstellt, wobei die Steuermittel geeignet sind, den Kammerreizschwellentest zu beenden, sobald der Reizverlust detektiert wird.

9. Der Programmierer gemäss Anspruch 8, in welchem N₃ ≤ 18 Zyklen.

10. Der Programmierer gemäss Anspruch 1, in welchem die Steuermittel ausserdem geeignet sind, die Durchführung wenigstens
- eines Testschritts (S₁₁) der Kontinuität der Schockspule,
- eines Testschritts (S₁₂) der Ladedauer eines Schockkondensators,
- getrennte Testschritte (S₄, S₁₄) der rechten und der linken Kammerreizschwelle,
zu steuern.

11. Der Programmierer gemäss Anspruch 1, in welchem die Anwenderschnittstelle Mittel (46) aufweist, um dem Anwender zu erlauben, vorläufig wenigstens eine spezifische Tätigkeit auszuwählen, die ohne Einsatz des Anwenders nach Durchführung der Testschritte sequentiell nacheinander durchzuführen sind.

12. Der Programmierer gemäss Anspruch 11, in welchem obengenannte spezifische Tätigkeiten der Gruppe gehören, die folgende Tätigkeiten einschliesst : Speichern des Testergebnisses im Implantat, Speichern der Testergebnisse im Programmierer, Drucken der Testergebnisse, im Anschluss an die Teste durchgeführte Aktivierung eines Diagnostikhilfemoduls.
